(19) Europäisches Patentamt
European Patent Office
Office européen des brevets

(11) Veröffentlichungsnummer : **0 406 643 B1**

(12) # EUROPÄISCHE PATENTSCHRIFT

(45) Veröffentlichungstag der Patentschrift : **08.12.93 Patentblatt 93/49**

(51) Int. Cl.$^5$ : **C08F 8/44,** A61K 6/00

(21) Anmeldenummer : **90111857.0**

(22) Anmeldetag : **22.06.90**

(54) **Gemischte, teilneutralisierte Salze von niederen Alkyl-Vinyl-Ether Maleinsäure Copolymeren und damit hergestellte Zahnprothesehaftmittel.**

(30) Priorität : **06.07.89 DE 3922170**

(43) Veröffentlichungstag der Anmeldung : **09.01.91 Patentblatt 91/02**

(45) Bekanntmachung des Hinweises auf die Patenterteilung : **08.12.93 Patentblatt 93/49**

(84) Benannte Vertragsstaaten : **DE ES FR GB IT**

(56) Entgegenhaltungen :
**EP-A- 0 014 736**
**EP-A- 0 289 713**
**DE-A- 2 250 731**
**GB-A- 420 589**
**US-A- 3 003 988**

(73) Patentinhaber : **Giulini Chemie GmbH**
**Giulinistrasse 2 Postfach 150 480**
**D-67029 Ludwigshafen (DE)**

(72) Erfinder : **Pelah, Zvi**
**P.O. Box 31 69**
**Savyon 56540 (IL)**
Erfinder : **Urmann, Ernat, Dr.**
**Limesstrasse 3**
**D-6700 Ludwigshafen (DE)**
Erfinder : **Kopp, Werner, Dr.**
**Schönbrückstrasse 1**
**D-6721 Harthausen (DE)**

**EP 0 406 643 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue gemischte, teilneutralisierte Salze von niederen Alkyl-Vinyl-Ether Maleinsäure Copolymeren und ihre Verwendung in Zahnprothesenhaftmitteln, die zur Fixierung von künstlichen Gebissen dienen.

Handelsübliche Haftmittel für Zahnprothesen werden als Pulver, Creme oder als Flüssigkeit angeboten. Ihre wesentliche Aufgabe ist es, für den idealen Sitz und die bestmögliche Haftung der Prothese zu sorgen. Der Mundspeichel spielt bei der Haftung eine wichtige Rolle, denn erst durch die Kombination zwischen Speichel und Haftmittel gelingt es, eine Verbindung zwischen der Prothese und dem Gaumen durch die Ausbildung von Kohäsions- und Adhäsionskräften herzustellen. Die Haftmittel müssen darum Stoffe enthalten, die mit dem Speichel quellbar sind und einen elastischen Film auf der Prothesenbasis bilden. Als Haftmittel eignen sich nur Substanzen, die eine Reihe von Anforderungen erfüllen, z.B. dürfen sie nicht schleimhautreizend sein und sie müssen toxikologisch unbedenklich sein, insbesondere im Hinblick auf Schwermetalle, da diese mit dem Speichel in den Magendarmtrakt gelangen könnten. Sie dürfen keine mikrobiologischen Verunreinigungen aufweisen und dürfen natürlich mit dem Prothesenmaterial chemisch nicht reagieren. Sie sollten frei von störendem Geruch bzw. Geschmack sein und schließlich müssen sie für eine möglichst lange Haftdauer sorgen, aber auch sicher und rückstandsfrei vom Gaumen und von der Prothese entfernbar sein.

Früher wurden natürliche Quellstoffe als Haftmittel verwendet. Es waren natürliche Polymere, z.B. Polysaccharide, die mit Wasser hochviskose Lösungen oder Suspensionen mit elastischem Verhalten bilden. In moderneren Formulierungen werden diese natürlichen Substanzen mit synthetischen Polymeren kombiniert oder synthetische Polymere alleine verwendet.

So findet in der DE-OS 19 09 209 das natürliche Polymere Traganth oder Karayaguumi in Kombination mit Natriummethylcellulose als klebende Komponente Verwendung. Das natürliche Polymer Traganth quillt im wässrigen Milieu des Mundes und kann erst in diesem Zustand die Haftung unterstützen. Die Größe der Haftkraft ist aber von der individuellen Speichelmenge im Mund abhängig, d. h. sie ist von Träger zu Träger verschieden stark ausgeprägt.

In der DE-OS 20 25 268 wird eine Dentalhaftcreme beschrieben, die als klebende Komponente Polyvinylacetat und Weichmacher enthält. Der Weichmacher erschwert jedoch die Entfernung der Haftcreme von der Prothese zu Reinigungszwecken und übt außerdem eine hautreizende Wirkung auf die Mundschleimhäute aus. Enthält diese Creme zu viel Weichmacher, dann wird sie außerdem zu weich und die Haftkraft ist selbst für normale Beanspruchung nicht ausreichend.

In der in der DE-OS 14 67 795 offenbarten Haftcreme findet als klebende Komponente Polyvinylpyrrolidon Verwendung. Ein großer Nachteil dieser Haftcreme besteht jedoch darin, daß die zur Geschmacksneutralisierung eingesetzten Zusatzstoffe, insbesondere Citratderivate den Geschmack negativ beeinflussen.

Die Herstellung und Verwendung der gemischten Ca/Na-Salze des Copolymeren Maleinsäureanhydrid/Methylvinylether ist in der US Patentschrift 3,003,988 beschrieben. Dieses pulverförmige Material eignet sich sehr gut als klebende Komponente in Haftmitteln. Es weist aber den entscheidenden Nachteil auf, daß merkliche Schwankungen in der Haftkraft auftreten können, die offenbar auf das Trocknungsverfahren des Salzes zurückzuführen sind.

In der DE-PS 21 33 709 sollen die in der US-A 3,003,988 erwähnten Nachteile beseitigt sein. In diesem Patent wird die Lehre erteilt, daß das pulverförmige Copolymersalz nur dann die beste Haftkraft aufweist, wenn es in einem Wirbelschichtofen getrocknet wird. Seine maximale Haftkraft ist erreicht, wenn die Feuchtigkeit in dem pulverförmigen Salz bei 10 % + 1 % liegt und diese auch konstant gehalten wird.

Die Herstellung des Grundpolymeren Maleinsäureanhydrid/Methylvinylether, das in den Patentschriften DE-OS 21 33 709 und US 3,003,988 zur Salzbildung eingesetzt wird, kann z.B. aus den US Patenten 2,782,182 oder 2,047,398 entnommen werden. Die radikalische Polymerisation der Monomeren Maleinsäureanhydrid und Methylvinylether wird in einem Lösungsmittel z. B. Benzol oder Methylenchlorid durchgeführt, wobei es nur bei der Verwendung von Benzol gelingt, ein Endprodukt mit einem ausreichend hohem Polymerisationsgrad zu erhalten. Nach der Trocknung enthält das so hergestellte Copolymere noch große Mengen an Benzol, das destillativ oder durch einfaches Trocknen nicht entfernbar ist. Deshalb kommen diese Polymere zur Herstellung der entsprechenden Ca/Na Salze nicht in Frage. In einer medizinisch-kosmetischen Anwendung wie z. B. in Prothesenhaftmitteln ist die Verwendung von solchen Materialien in einigen Ländern sogar gesetzlich verboten.

Ein verbessertes Verfahren zur Herstellung der entsprechenden benzolfreien Copolymeren, das von der Anmelderin entwickelt wurde, ist z.B. aus der DE-PS 37 12 265 bekannt. Ein solches Material ist besonders zur Herstellung der erfindungsgemäßen neuen Al/Ca/Na-Salze geeignet, und ebenso für die in der US-A 3,003,988 und DE-PS 21 33 709 genannten Ca/Na Salze.

In der EP A2 0 265 916 wird über neuere Zahnprothesenhaftmittel berichtet, die neben den Ca/Na-Salz

des MSA/MVE-Copolymeren noch Zink- bzw. Strontiumsalze dieses Copolymeren enthalten. Das Zinksalz wird bevorzugt in dem entsprechenden Haftmittel eingesetzt. Enthält das Haftmittel entweder das Zink- oder das Strontiumsalz, und auch das Calcium/Natriumsalz des MSA/MVE-Copolymeren, soll die Haftwirkung erheblich besser sein, als in Formulierungen, die das Ca/Na-Salz allein enthalten. Ein großer Nachteil dieses Haftmittels ist jedoch, daß es die toxischen Metalle Zink und Strontium enthält. Außerdem muß das nach Beispiel 1 hergestellte Salz 16 bis 18 Stunden lang bei 70 ° C getrocknet werden. Eine derart lange Trocknungszeit ist jedoch sehr unwirtschaftlich, ebenso wie die Tatsache, daß in dem Herstellungsprozeß dieser Zink- bzw. Strontiumsalze in stark verdünnter, wäßriger Suspension gearbeitet wird.

Es stellte sich daher die Aufgabe klebende Komponenten für Zahnprothesenhaftmittel zu finden, die einerseits eine erheblich verbesserte Haftkraft aufweisen, andererseits keine physiologisch bedenklichen Schwermetalle enthalten und außerdem sich ökonomisch herstellen lassen.

Die Aufgabe konnte durch die Bereitstellung von gemischten, teilneutralisierten Salzen von niederen Alkyl-Vinylether Maleinsäure Copolymeren gelöst werden, die die folgende Struktureinheit aufweisen:

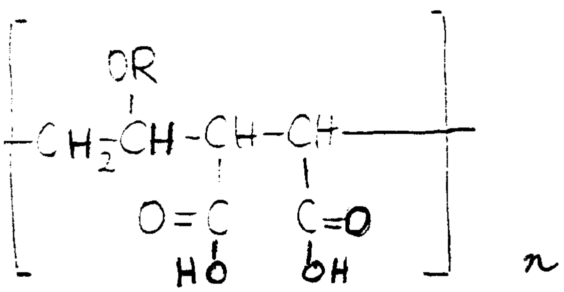

Hier bedeutet R einen Alkylrest mit 1 bis 4 Kohlenstoffatomen, n eine ganze Zahl größer als 0,wobei n so gewählt werden muß, daß die spez. Viskosität des korrespondierenden Anhydrids bei 2,6 bis 3,5 liegt. 30 bis 95 % der Carboxylgruppen sind darin durch Aluminium, Calcium und Natrium neutralisiert. Der pH-Wert der Salze liegt zwischen 6,5 und 7,5. Das Al : Ca-Verhältnis im Salz beträgt zwischen 1:1 und 1:4.

Werden die Carboxylgruppen nur mit dem Aluminiumkation neutralisiert, wobei R=Methyl ist, so kann dieses Al-Salz gemäß Beispiel 4 als Ausgangsverbindung zur Herstellung der erfindungsgemäßen Al/Ca/Na Salze eingesetzt werden.

Die neuen Al-haltigen Salze eignen sich zur Herstellung von Zahnprothesenhaftmittel. Weitere hierzu verwendete Zusätze, wie z. B. Vaseline, Aerosil u.a. sind im Stand der Technik bekannte Substanzen. Zahnprothesenhaftmittel, die das neue Al-haltige Salz des MSA/MVE-Copolymeren als klebende Komponente enthalten, weisen eine erheblich verbesserte und konstante Haftkraft, verglichen mit den handelsüblichen Haftmittelformulierungen, auf.

Ein entscheidender Vorteil des erfindungsgemäßen Al-Salzes besteht darin, daß das Metallionen $Al^{3+}$ physiologisch weniger bedenklich ist, als die entsprechenden Zink- oder Strontiumsalze gemäß EP A2 0 265 916. Es ist außerdem bekannt, daß Aluminiumsalze bakterizide Wirkung haben. Diese ist besonders im Mundbereich sehr vorteilhaft, weil sich hier ständig viele Keime befinden.

Die neuen Salze sind durch mehrere Verfahren gleichermaßen gut erhältlich. Als Ausgangsprodukt für die Herstellung eines erfindungsgemäßen Salzes, z.B. des Al/Ca/Na-Salzes kann das Ca/Na Salz eingesetzt werden, dessen Herstellung z.B. in der US-Patentschrift 3 003 988 beschrieben ist. Das dort erwähnte Salz ist ein teilneutralisiertes Ca/Na Salz des MSA/AVE Copolymeren in dem R=Methyl ist und das einem Neutralisationsgrad von 64 - 80 % und einem Ca:Na Verhältnis von 2,5 - 4 : 1 (Beisp. 6 und 7) hat. Es weist in der Regel pH-Werte zwischen 6 und 7,5 auf. Nach der US-Patentschrift können aber auch alle anderen Ca/Na Salze eingesetzt werden, die in den Bereich eines Ca:Na Verhältnisses von 1:1 bis 5:1 fallen.

Weiterhin können die Salze gemäß DE-PS 21 33 709 eingesetzt werden, bei denen ein Teil der nicht an Natrium oder Calcium gebundenen Carboxylgruppen verestert ist.

Ferner ist es möglich, die erfindungsgemäßen Salze auch aus dem Copolymeren Maleinsäureanhydrid/Methylvinylether (MSA/MVE) herzustellen, das z.B. im Handel unter dem Namen Gantrez AN 169$^R$ der Fa GAF erhältlich ist. Es handelt sich dabei um ein hochmolekulares Copolymer mit einer spezifischen Viskosität von 2,6 bis 3,5. Es ist auch möglich von Derivaten des Gantrez AN 169$^R$ auszugehen, bei denen alle Anhydridgruppen hydrolysiert sind. Diese ebenfalls hochmolekulare Säure ist im Handel unter dem Namen Gantrez S 97$^R$ erhältlich. Die nach dem neuen Verfahren gemäß DE-PS 37 12 265 erhältlichen MSA/MVE Copolymeren sind zur Herstellung der erfindungsgemäßen Salze besonders gut geeignet. Gleich von welchem Produkt man ausgeht, es ist wichtig die Zusammensetzung, insbesondere das Ca/Na Verhältnis und den Neutralisationsgrad zu kennen.

Die übrigen zur Herstellung der erfindungsgemäßen Salze benötigten Ausgangsverbindungen sind im Falle des Anhydrids und der Säure bekannte, wohldefinierte Substanzen, z.B. Natronlauge, Calciumhydroxid u.a.. Als Aluminiumkomponente sind z. B. geeignet: Aluminium-triisopropylat, in dem Aluminium als dreiwertiges Kation enthalten ist. Diese Verbindung ist im Handel erhältlich. Auch ein frisch gefälltes noch aktives Aluminiumhydroxid mit einem $Al_2O_3$-Gehalt von z.B. 70 % kann eingesetzt werden. Prinzipiell kann man alle Al-haltigen Salze verwenden, die $Al^{3+}$ Kationen enthalten. Allerdings sollten solche Salze von Aluminium, die toxische oder hautreizende Anionen haben, z.B. Chlorid, Nitrat, Sulfat u.a. nicht eingesetzt werden, bzw. wenn sie doch verwendet werden, sollten diese nach der Herstellung des Endproduktes sorgfältig entfernt werden.

Die Herstellung der erfindungsgemäßen Salze wird in den folgenden Beispielen näher erläutert:

Beispiel 1

2000 ml Isopropanol werden in einem Gefäß mit Rührer vorgelegt und 562 g Maleinsäureanhydrid/Methylvinylether Copolymer mit einer spezifischen Viskosität von 2,6 bis 3,5 (erhältlich von der Fa. GAF unter dem Namen Gantrez AN 169) und 133 g Calciumhydroxid sowie 61,8 g Al-triisopropylat hinzugefügt. Diese Mischung wird eine halbe Stunde gut gerührt, wonach man 36 g Natriumhydroxid- Schuppen, gelöst in 100 ml Wasser unter Rühren innerhalb von 30-60 Minuten zulaufen läßt. Die Temperatur erhöht sich während der einsetzenden Reaktion auf 36 bis 38 °C und wird durch Heizung weiter bis auf 50 °C angehoben. Die Mischung wird bei dieser Temperatur so lange gerührt, bis der pH den Wert von 7 erreicht hat Nach dem Erkalten wird die Suspension abgenutscht und nach dem Trocknen bei einer Temperatur von 100 °C gemahlen und auf < 50 μ gesiebt.

Man erhält ein 1:4:1 Al/Ca/Na Salz mit einem Anteil von freien und veresterten Carboxylgruppen von 25 %.

Beispiel 2

2000 ml Isopropylalkohol werden in dem Gefäß des Beispiels 1 vorgelegt und unter Rühren 609 g Maleinsäure/Methylvinylether Copolymer, hochmolekular (z. B. Gantrez S-97[R] der Fa. GAF) suspendiert und ebenfalls unter Rühren 104 g $Ca(OH)_2$ und 51 g eines reaktiven Aluminiumhydroxids ($Al_2O_3$-Gehalt: 70 %) zugegeben. Anschließend wird die Temperatur auf 43 - 45 °C angehoben und innerhalb einer halben Stunde 56 g NaOH in 100 ml Wasser hinzugefügt. Die Temperatur, die noch etwas ansteigt, wird bei 50 °C konstant gehalten bis der pH-Wert der Suspension auf 7 gefallen ist. Anschließend wird abgenutscht, mit etwas Isopropanol gewaschen und bei 70 - 80 °C getrocknet. Das Produkt wird anschließend gemahlen und auf < 50 μ gesiebt.

Man erhält ein 1,5:2:1 Al/Ca/Na-Salz mit einem Neutralisationsgrad von 90 %.

Beispiel 3

3000 ml Isopropylalkohol werden vorgelegt und unter Rühren 683 g eines 3,5:1 Ca/Na Salzes von MSA/MVE Copolymeren mit einem Neutralisationsgrad von 54 % suspendiert. Unter intensivem Rühren werden dann 114 g Aluminiumtriisopropylat hinzugefügt und die Temperatur auf 60 °C angehoben. Der pH-Wert sinkt dabei von anfangs 9-10 auf den Wert von 7, wobei die Umsetzung durch Abkühlen und Absaugen des Isopropanols beendet wird. Der Filterkuchen wird in der in Beispiel 1 und 2 beschriebenen Weise aufgearbeitet.

Man erhält ein 2:3,5:1 Al/Ca/Na-Salz mit einem Neutralisationsgrad von 78 %.

Beispiel 4

1000 ml Isopropanol werden vorgelegt und unter Rühren 200 g eines Maleinsäure/Methylvinylether-Copolymers (hochmolekular, z .B: Gantrez S-97[R] der Fa. GAF) suspendiert. Unter intensivem Rühren werden dann 114 g Aluminiumtriisopropylat zugegeben und die Mischung auf 60 °C erwärmt. Unter Rühren wird die Temperatur für 1 Stunde konstant gehalten. Die Suspension wird nach dem Erkalten abgenutscht, bei 70-80 °C im Trockenschrank getrocknet, gemahlen und auf< 50 μ gesiebt.

Man erhält ein Al- Salz des MSA/MVE Copolymeren mit einem Neutralisationsgrad von 73 %.

100 g dieses Salzes werden mit 225 g eines 3,5:1 Ca/Na-Salzes des gleichen Copolymeren (80 % neutralisiert), das ebenfalls gemahlen und auf < 50 μ gesiebt wurde, intensiv vermischt. Man erhält ein 2:3,5:1 Al/Ca/Na-Salz des MSA/MVE Copolymeren mit einem durchschnittlichen Neutralisationsgrad von 78 %.

Es ist sowohl in seinem Ca, Na-und Aluminiumgehalt, als auch im Neutralisationsgrad mit dem im Beispiel 3 hergestellten Produkt identisch. Lediglich in der Kationenverteilung innerhalb des Polymeren unterscheiden

sich beide Produkte.

Beispiel 5

420 l Isopropanol werden in einem 1000 l Horizontalmischer (Draismischer) vorgelegt und 120,5 kg eines 3,5:1 Ca/Na- Salzes mit einem Neutralisationsgrad von 67,5 % suspendiert. Ebenfalls unter Rühren werden 12,3 kg Aluminiumtriisopropylat zugegeben und nach 30 Minuten Mischzeit die Temperatur auf 55 °C angehoben. Nach einer Stunde Rührzeit bei dieser Temperatur - der pH-Wert sollte jetzt zwischen 7 und 7,5 liegen - wird mit dem Abdestillieren des Isopropanols begonnen.

Die Wirbelschichttrocknung wird unter Vakuum (200-250 mbar) solange fortgesetzt, bis das gesamte Isopropanol entfernt ist. Die Produkttemperatur sollte dabei 70 °C nicht überschreiten.

Das derart gleichmäßig getrocknete Produkt zerfällt in der Regel zu einem feinem Pulver und kommt anschließend bis zu einem Feinheitsgrad von weniger als 50 μ zur Vermahlung.

Man erhält ein 1:3,5:1 Al/Ca/Na Salz von MSA/MVE Copolymer mit einem Neutralisationsgrad von 82,5 %.

Beispiel 6

Es werden zwei Haftcremeformulierungen hergestellt, die sich lediglich durch die Copolymerkomponente unterscheiden. In der Formulierung A wird ein im Stand der Technik bekanntes Ca/Na-Salz des Copolymeren MSA/MVE verwendet, in der Formulierung B das erfindungsgemäße Al/Ca/Na-Salz des Copolymeren MSA/MVE:

| Zusammensetzung: | [Angaben in Gew. %] A | B |
|---|---|---|
| Mineralöl | 37 | 37 |
| Vaseline | 12 | 12 |
| Aerosil | 1 | 1 |
| 3,5:1 Ca/Na Salz von MSA/MVE Copolymer (78 % neutralisiert) | 50 | - |
| 2:3,5:1 Al/Ca/Na Salz von MSA/MVE Copolymer (78 % neutralisiert) | - | 50 |
| | 100 | 100 |

Beispiel 7

Die Klebekraft der Haftcremeformulierungen aus dem Beispiel 6 wird mit Hilfe der in der DE-PS 21 33 709 beschriebenen Apparatur getestet. Es werden dabei 0,5 g der Haftcreme steigende Wassermengen (jeweils 0,5 g mehr) zugesetzt:

| | ohne Wasser | + 0,5 g Wasser | + 1 g Wasser | + 1,5 g Wasser | + 2 g Wasser |
|---|---|---|---|---|---|
| Klebekraft von 6A [$kg/cm^2$] | 1,27 | 2,19 | 4,02 | 3,87 | 2,91 |
| Klebekraft von 6B [$kg/cm^2$] | 1,29 | 3,52 | 4,61 | 4,74 | 3,85 |

## Patentansprüche

1. Gemischte, teilneutralisierte Salze von niederen Alkyl-Vinyl-Ether-Maleinsäureanhydrid Copolymeren (AVE/MSA), die die folgende Struktureinheit aufweisen,

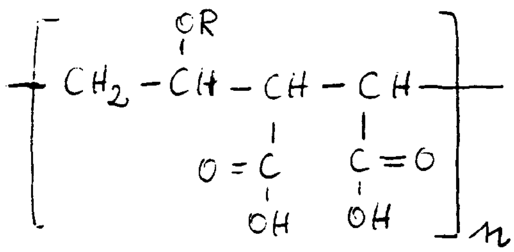

worin R einen Alkylrest mit 1 bis 4 Kohlenstoffatomen bedeutet, 30 bis 95 % der Carboxylgruppen mit Aluminium, Calcium und Natrium neutralisiert sind und der pH-Wert der Salze zwischen 6,5 und 7,5 liegt und n eine ganze Zahl größer Null ist, wobei n so gewählt werden muß, daß die spezifische Viskosität des korrespondierenden Anhydrids bei 2,6 bis 3,5 liegt, und das Al:Ca Verhältnis zwischen 1:1 und 1:4 liegt.

2. Verbindung nach Anspruch 1, dadurch gekennzeichnet, daß R Methyl ist.

3. Verbindung nach Anspruch 1, dadurch gekennzeichnet, daß zur Neutralisierung das Kation Aluminium, verwendet wird und R Methyl ist.

4. Zahnprothesenhaftmittel enthaltend die Verbindungen nach den Ansprüchen 1 bis 3.

5. Verwendung der Verbindungen gemäß den Ansprüchen 1 bis 3 in Zahnprothesenhaftmitteln.

## Claims

1. Mixed, partially neutralised salts of lower alkylvinyl-ether/maleic acid anhydride copolymers (AVE/MSA) which contain the following structural unit

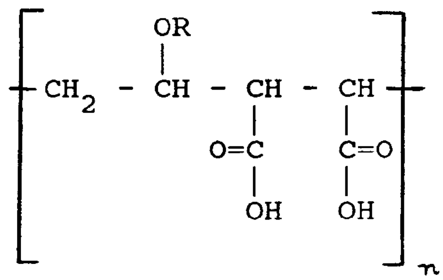

wherein R denotes a $C_{1-4}$-alkyl group, 30 to 95% of the carboxyl groups are neutralised with aluminium, calcium and sodium and the pH of the salts is between 6.5 and 7.5 and n is an integer greater than zero, whilst n must be chosen so that the specific viscosity of the corresponding anhydride is from 2.6 to 3.5, and the Al:Ca ratio is between 1:1 and 1:4.

2. Compound according to claim 1, characterised in that R is methyl.

3. Compound according to claim 1, characterised in that the cation aluminium is used for the neutralisation and R is methyl.

4. Denture adhesive containing the compounds according to claims 1 to 3.

5. Use of the compounds according to claims 1 to 3 in denture adhesives.

## Revendications

1. Sels mixtes, partiellement neutralisés de copolymères d'anhydre maleique et d'éthers vinyliques-alkyliques inférieurs, présentant l'unité de structure suivante:

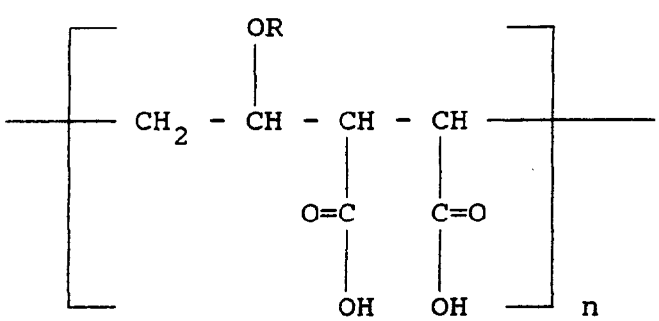

dans laquelle R représente un radical ou reste alkyle à 1 à 4 atomes de carbone, 30 à 95 % des groupes carboxyle sont neutralisés par de l'aluminium, du calcium et du sodium, la valeur de pH des sels est comprise entre 6,5 et 7,5 et n represente un nombre entier supérieur à zéro, n étant choisi tel que la viscosité spécifique de l'anhydride correspondant soit de 2,6 à 3,5 et que le rapport Al:Ca soit compris entre 1:1 et 1:4.

2. Composé suivant la revendication 1, caractérisé en ce que R représente un groupe méthyle.

3. Composé suivant la revendication 1, caractérisé en ce que le cation aluminium est utilisé pour la neutralisation et R représente un groupe méthyle.

4. Adhésif pour prothèse dentaire contenant les composés selon les revendications 1 à 3.

5. Utilisation des composés selon les revendications 1 à 3 dans des adhésifs pour prothèse dentaire.